# EUROPÄISCHE PATENTANMELDUNG

(11) **EP 3 808 381 A1**
(43) Veröffentlichungstag der Anmeldung: **21.04.2021**
(21) Anmeldenummer: 19203859.4
(22) Anmeldetag: 18.10.2019
(51) Int. Cl.: A61K 49/06, C07C 229/00

(54) **EISEN(III)-KOMPLEXE MIT NEUEN KONTRASTMITTELEIGENSCHAFTEN FÜR DIE MAGNETRESONANZTOMOGRAPHIE**

(71) Anmelder: Charité - Universitätsmedizin Berlin, 10117 Berlin (DE)
(72) Erfinder: Schellenberger, Eyk, 10115 Berlin (DE); Hauptmann, Ralf, 12203 Berlin (DE); Häckel, Akvile, 06886 Lutherstadt Wittenberg (DE); Xie, Jing, 10555 Berlin (DE); Schnorr, Jörg, 16515 Oranienburg (DE); Hamm, Bernd, 12207 Berlin (DE)
(74) Vertreter: Gulde & Partner

(57) **Zusammenfassung**

Die Erfindung stellt neuartige Eisen(III)-komplexe der Formeln (1) und (2) bereit. Ferner werden entsprechende Liganden der Formeln (L1) und (L2) zur Herstellung der Eisen(III)-komplexe offenbart.

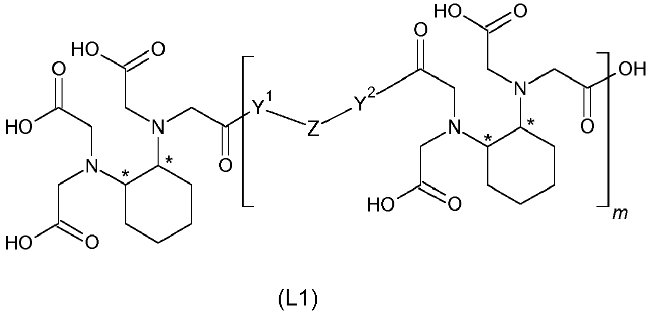

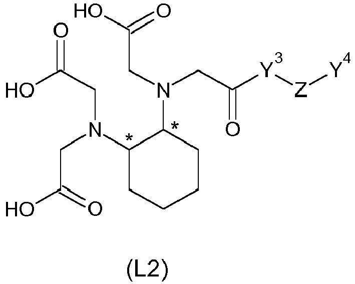

## Beschreibung

Die Erfindung betrifft neuartige Eisen(III)-komplexe, die sich insbesondere als Kontrastmittel für die Magnetresonanztomographie eignen. Ferner werden Liganden zur Herstellung dieser Eisen(III)-komplexe offenbart.

### Technologischer Hintergrund

Niedermolekulare Gadolinium-basierte Kontrastmittel (GBCA) haben sich als Standard zur Verbesserung der Aussagekraft von Magnetresonanztomographie (MRT)-Untersuchungen in allen Bereichen der Medizin durchgesetzt. In Deutschland wurden im Jahr 2015 über 10,8 Mio. Magnetresonanztomographie (MRT)-Untersuchen durchgeführt (de.statista.com). Bei etwa einem Drittel dieser MRT-Untersuchungen werden dabei GBCA für T1-gewichtete Bildgebungssequenzen intravenös appliziert, wodurch verbesserte Diagnosen bei akuten und chronischen Entzündungen, Tumoren, Gefäßneubildungen usw. ermöglicht werden. Dabei beschleunigen GBCA sowohl die T1-Relaxation (Spin-Gitter-Relaxation) als auch die T2-Relaxation (Spin-Spin-Relaxation), wobei die T1-Zeitverkürzung zu einer diagnostisch nutzbaren MRT-Signalerhöhung in entsprechend T1-gewichteten Bildgebungssequenzen führt.

Freie Gadolinium-Ionen sind toxisch (z.B. dos Remedios CG. Lanthanide ion probes of calcium-binding sites on cellular membranes. Cell Calcium. 1981; 2(1):29-51 und Bower DV et al. Gadolinium-Based MRI Contrast Agents Induce Mitochondrial Toxicity and Cell Death in Human Neurons, and Toxicity Increases With Reduced Kinetic Stability of the Agent. Investigative Radiology. 2019; 54(8):453-463). Aufgrund ihres im Vergleich zu Calcium-Ionen chemisch ähnlichen Verhaltens können diese beispielsweise in der Leber und im Knochensystem eingebaut werden und über Jahre dort verbleiben. Da die lonenradien von Calcium und Gadolinium ähnlich sind, kann Gadolinium als Calciumantagonist wirken und beispielsweise die Kontraktilität des Myokards und das Gerinnungssystem hemmen (Darrah TH et al. Incorporation of excess gadolinium into human bone from medical contrast agents. Metallomics. 2009; 1(6):479-488). Obwohl Gadolinium-Ionen toxisch sind, wurden GBCA mit der Maßgabe zugelassen, dass sie in den zugelassenen Pharmaka fest in Komplexbildnern, beispielsweise DTPA (Diethylentriaminpentaessigsäure) und DOTA (1,4,7,10-Tetraazacyclododecan-1,4,7,10-tetraessigsäure), eingebunden sind und zusammen mit diesen sehr schnell und vollständig vor allem durch die Niere ausgeschieden werden.

Allerdings hat sich gezeigt, dass die Ausscheidung unter Umständen nicht vollständig erfolgt. So wurde vor etwa 12 Jahren die sogenannte nephrogene systemische Fibrose als schwere Nebenwirkung unvollständig ausgeschiedener Gadolinium-Kontrastmittel erkannt, die Patienten mit eingeschränkter Nierenfunktion verabreicht wurden (Grobner T. Gadolinium - a specific trigger for the development of nephrogenic fibrosing dermopathy and nephrogenic systemic fibrosis? Nephrol Dial Transplant. 2006; 21(4):1104-1108 und Idee JM et al. The role of gadolinium chelates in the mechanism of nephrogenic systemic fibrosis: A critical update. Crit Rev Toxicol. 2014; 44(10):895-913). Eine andere tierexperimentelle Studie zeigte, dass nach Gabe von GBCA in Hautbiopsien von Ratten Gadolinium noch nach einem Jahr nachgewiesen werden konnte (Pietsch H et al. Long-term retention of gadolinium in the skin of rodents following the administration of gadolinium-based contrast agents. Eur Radiol 2009; 44(4):226-233). Das Ausmaß der Gadolinium-Anreicherung erwies sich dabei abhängig von der Art der Komplexbildner, insbesondere der Stabilität der Gadolinium-Komplexe.

Besonders bei wiederholter Gabe von niedermolekularen Kontrastmitteln, wie sie beispielsweise zum Brusttumor-Screening oder zu Verlaufskontrollen bei multipler Sklerose notwendig sind, wurden Ablagerungen im Hirngewebe von gesunden Personen gefunden (siehe beispielsweise McDonald RJ et al. Intracranial Gadolinium Deposition after Contrast-enhanced MR Imaging. Radiology 2015; 275(3):772-782). Mittels ICP-MS wurden in Proben von verstorbenen Patienten auch Gadolinium-Ablagerungen von stabileren makrozyklischen GBCA besonders in Knochen und im Hirn gefunden (Murata N et al. Macrocyclic and Other Non-Group 1 Gadolinium Contrast Agents Deposit Low Levels of Gadolinium in Brain and Bone Tissue: Preliminary Results From 9 Patients With Normal Renal Function. Invest Radiol 2016; 51(7):447-453).

In der Vergangenheit konnte gezeigt werden, dass niedermolekulare Eisenkomplexe (besonders Fe-DTPA, Fe-*t*CDTA (*t*CDTA = trans-Cyclohexandiamintetraessigsäure) als Kontrastmittel für die MRT genutzt werden können. Die Relaxivitäten dieser Eisenkomplexe sind zwar im Vergleich zu den GBCA geringer, aber durch höhere Dosierungen lässt sich dieser Nachteil kompensieren (siehe beispielsweise White DL et al. IRON (III) COMPLEXES AS MRI CONTRAST AGENTS. Proc. Int. Soc. Magn. Reson. Med. 1985; 1985(S2):906-909).

Aufgrund der großen Menge an körpereigenem Eisen (ca. 4 g für einen Erwachsenen) und der dafür vorhanden Systeme für Aufnahme, Speicherung und Transport ist davon auszugehen, dass die Langzeittoxizität für Eisenkomplexe geringer sein sollte als für GBCA. Außerdem werden Eisenoxidnanopartikel (verwendbar als Kontrastmittel oder für Eisenersatztherapie) allgemein gut vertragen, obwohl diese, im Gegensatz zu den fast vollständig ausgeschiedenen niedermolekularen Kontrastmitteln, nach intravenöser Gabe nahezu vollständig im Körper verbleiben.

Es wurde bereits gezeigt, dass Eisenkomplexe sich auch für typische aktuelle Anwendungen, wie z. B. die dynamische kontrastmittelgestützte MRT (DCE-MRI) eignen, die beispielsweise in der Brusttumordiagnostik eingesetzt wird (Boehm-Sturm P et al., Low-Molecular-Weight Iron Chelates May Be an Alternative to Gadolinium-based Contrast Agents for T1-weighted Contrast-enhanced MR Imaging. Radiology 2017; 286(2):537-546).

US 5362475 A beschreibt unter anderem die Herstellung Eisen-basierter Kontrastmittel, konkret dem Natrium- oder N-Methylglucaminsalz eines Eisen(III)-komplexes mit tCDTA-Ligand.

Gestin et al. (Nucl. Med. Biol., 1993; 20(6),755-762) beschreibt unter anderem die Synthese von N-[Methyl(2-aminoethyl)carbamid]-trans-1,2-diaminocyclohexan-N,N',N'-triessigsäure als ein Zwischenprodukt neuartiger bifunktionaler Chelatbildner für Indium-markierte Antikörper.

Es besteht somit ein anhaltender Bedarf nach alternativen, Gadolinium-freien Kontrastmitteln. Ferner wäre es von wünschenswert, wenn Kontrastmittel beispielsweise ein pH-Wertabhängiges Resonanzverhalten zeigen würden, so dass sich neue diagnostische Möglichkeiten ergeben.

### Zusammenfassung der Erfindung

Die Erfindung stellt neuartige Eisen(III)-komplexe der Formeln (1) und (2) gemäß Anspruch 1 bereit: 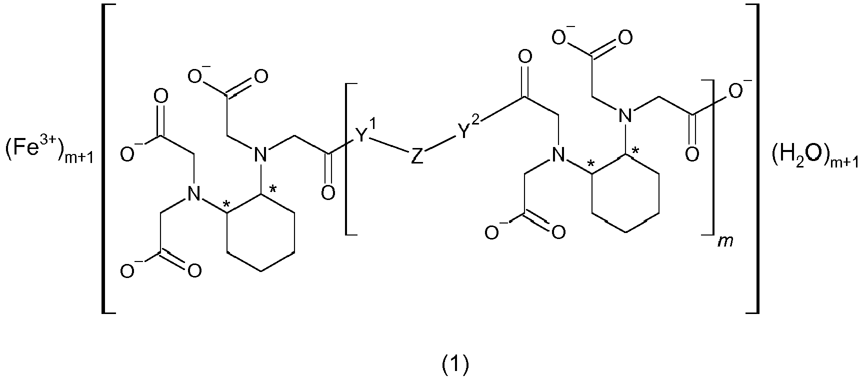 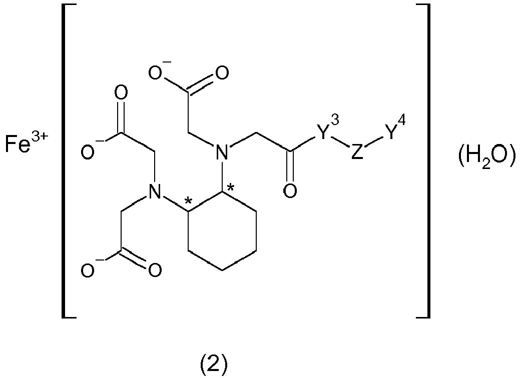 mit
m = 1 bis 5;
Y¹ und Y² unabhängig voneinander ausgewählt aus der Gruppe umfassend O, NH und NR, wobei R für C1-C15 Alkyl oder C3-C10 Cycloalkyl steht;
Y³ ausgewählt aus der Gruppe umfassend O, NH und NR, wobei R für C1-C15 Alkyl oder C3-C10 Cycloalkyl steht;
Y⁴ ausgewählt aus der Gruppe umfassend OH, OR, NH₂, NHR und NR⁵R⁶, wobei R, R5 und R6 für C1-C15 Alkyl oder C3-C10 Cycloalkyl stehen sowie R⁵ und R⁶ unabhängig voneinander ausgewählt sind;
Z bedeutet eine Brücke gemäß einer der Formeln (3) bis (10): 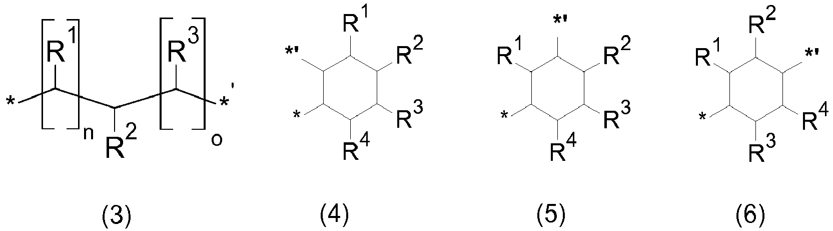 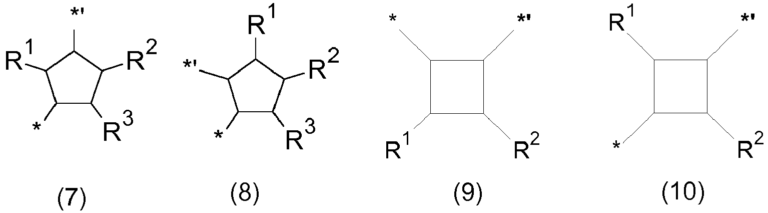 mit
   n = 0 bis 4;
   o = 0 bis 4;
   * und *' den Anbindungsstellen der Brücke Z an Y¹ und Y² der Formel (1) beziehungsweise Y³ und Y⁴ der Formel (2); und
   R¹, R², R³ und R⁴ unabhängig voneinander ausgewählt aus der Gruppe umfassend H, C1-C15 Alkyl und C3-C10 Cycloalkyl.

Weitere Aspekte der Erfindung liegen in der Verwendung der Eisen(III)-komplexe der Formeln (1) oder (2) in in-vivo-diagnostischen Verfahren, insbesondere als Kontrastmittel für die Magnetresonanztomographie, sowie in der Bereitstellung eines Kontrastmittels für Magnetresonanztomographie, das die genannten Eisen(III)-komplexe enthält.

Ferner werden durch die Erfindung als Zwischenprodukt neuartige Liganden der Formeln (L1) oder (L2) bereitgestellt, die mit Eisen(III) zu den obig beschriebenen Komplexen umsetzbar sind: 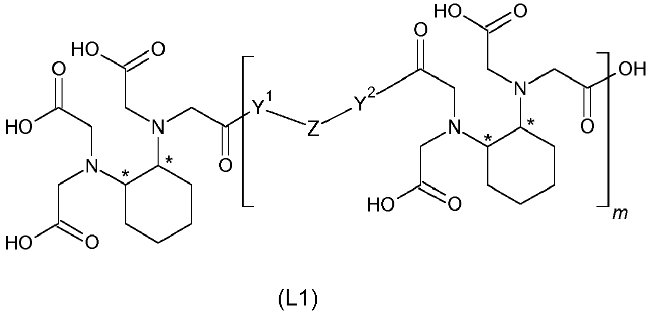 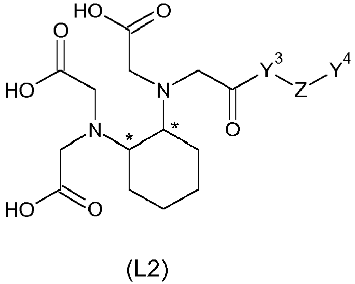 mit
m = 1 bis 5;
Y¹ und Y² unabhängig voneinander ausgewählt aus der Gruppe umfassend O, NH und NR, wobei R für C1-C15 Alkyl oder C3-C10 Cycloalkyl steht;
Y³ ausgewählt aus der Gruppe umfassend O, NH und NR, wobei R für C1-C15 Alkyl oder C3-C10 Cycloalkyl steht;
Y⁴ ausgewählt aus der Gruppe umfassend OH, OR, NH₂, NHR und NR⁵R⁶, wobei R, R⁵ und R⁶ für C1-C15 Alkyl oder C3-C10 Cycloalkyl stehen sowie R⁵ und R⁶ unabhängig voneinander ausgewählt sind;
Z bedeutet eine Brücke gemäß einer der Formeln (3) bis (10): 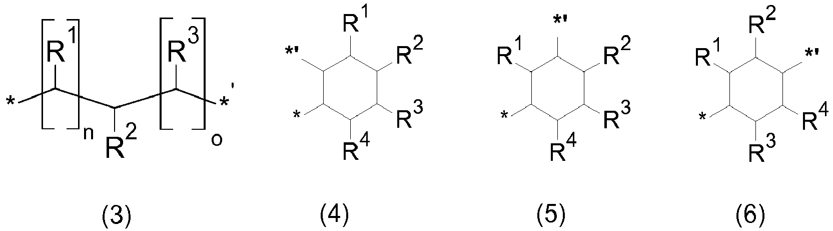 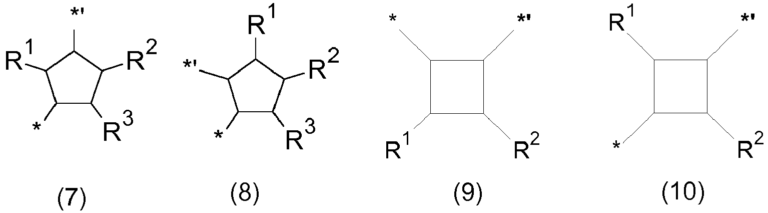 mit
   n = 0 bis 4;
   o = 0 bis 4;
   * und *' bedeuten Anbindungsstellen der Brücke Z an Y¹ und Y² der Formel (1) beziehungsweise Y³ und Y⁴ der Formel (2); und
   R¹, R², R³ und R⁴ unabhängig voneinander ausgewählt aus der Gruppe umfassend H, C1-C15 Alkyl und C3-C10 Cycloalkyl;
   ausgenommen den Liganden der Formel (L2) mit Z gleich *-CH₂CH₂-*'.

Weitere bevorzugte Ausgestaltungen der Erfindung ergeben sich aus der nachfolgenden Beschreibung.

### Kurzbeschreibung der Figuren

Die Erfindung wird nachfolgend anhand von Ausführungsbeispielen und einer dazugehörigen Zeichnung näher erläutert. Die einzige Figur zeigt:

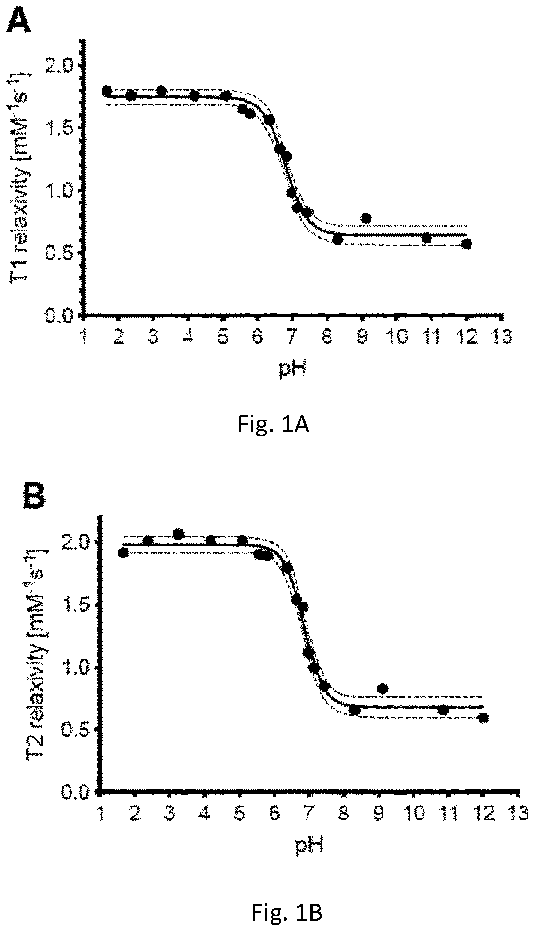

- Fig.1: pH-Abhängigkeit der T1- und T2-Relaxivität des Eisen(III)-komplexes (2-1)

### Detaillierte Beschreibung der Erfindung

Die Erfindung stellt neuartige Eisen(III)-komplexe der Formeln (1) und (2) gemäß Anspruch 1 bereit: 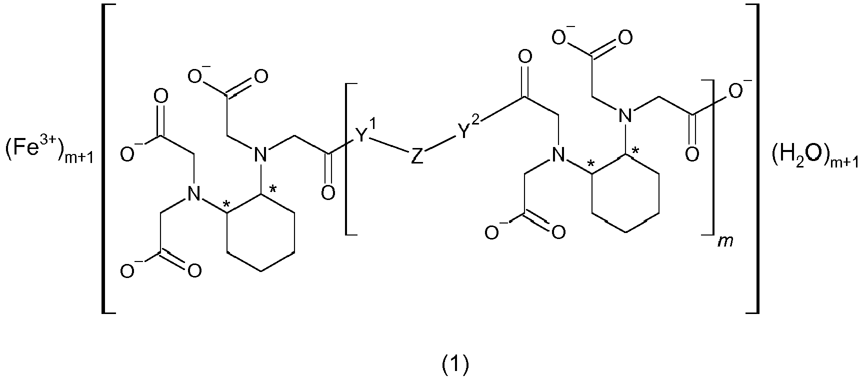 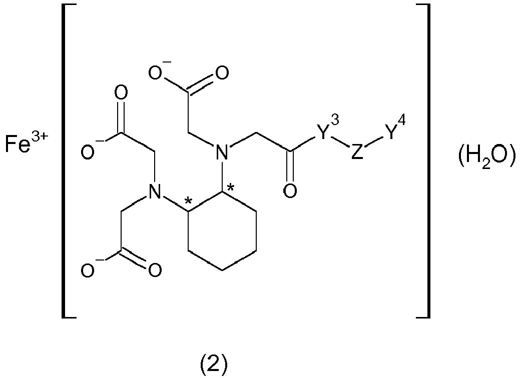 mit
m = 1 bis 5;
Y¹ und Y² unabhängig voneinander ausgewählt aus der Gruppe umfassend O, NH und NR, wobei R für C1-C15 Alkyl oder C3-C10 Cycloalkyl steht;
Y³ ausgewählt aus der Gruppe umfassend O, NH und NR, wobei R für C1-C15 Alkyl oder C3-C10 Cycloalkyl steht;
Y⁴ ausgewählt aus der Gruppe umfassend OH, OR, NH₂, NHR und NR⁵R⁶, wobei R, R⁵ und R⁶ für C1-C15 Alkyl oder C3-C10 Cycloalkyl stehen sowie R⁵ und R⁶ unabhängig voneinander ausgewählt sind;
Z bedeutet eine Brücke gemäß einer der Formeln (3) bis (10): 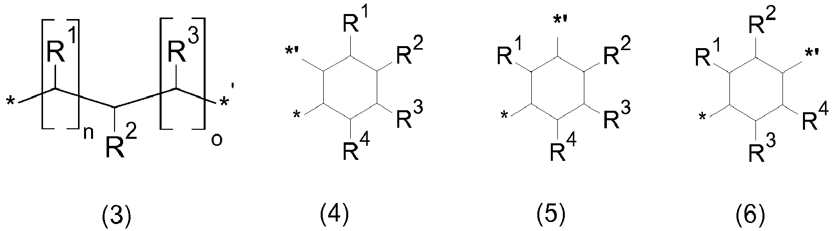 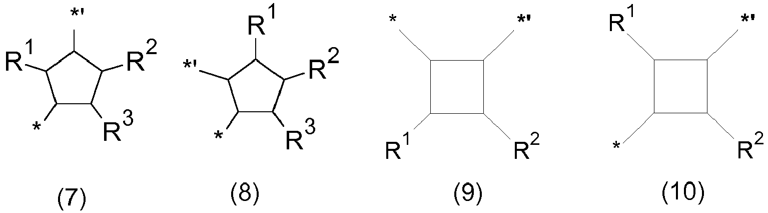 mit
   n = 0 bis 4;
   o = 0 bis 4;
   * und *' bedeuten Anbindungsstellen der Brücke Z an Y¹ und Y² der Formel (1) beziehungsweise Y³ und Y⁴ der Formel (2); und
   R¹, R², R³ und R⁴ unabhängig voneinander ausgewählt aus der Gruppe umfassend H, C1-C15 Alkyl und C3-C10 Cycloalkyl.

Die beiden Substituenten an den mit * markierten asymmetrischen Kohlenstoffatomen der Formeln (1) oder (2) können prinzipiell eine voneinander unabhängige Stereochemie aufweisen. Vorzugsweise sind die beiden Substituenten jedoch an den mit * markierten asymmetrischen Kohlenstoffatomen der Formeln (1) oder (2) trans-ständig angeordnet.

Die Brücken der Formeln (4) bis (10) weisen als Grundstruktur eine 4, 5 oder 6 Kohlenstoffatome aufweisende Ringstruktur auf. Die Stereochemie der einzelnen Kohlenstoffatome, die die Ringstruktur bilden, ist vorliegend nur von untergeordneter Bedeutung, also beliebig. Ebenso verhält es sich, wenn die Kohlenstoffatome der Brücke gemäß Formel (3) asymmetrisch sind. Sind in Formel (3) beispielsweise n + o = 0, so bedeutet dies, dass Z einer Methylen-Gruppe mit einem R²- und einem H-Substituenten entspricht und entweder (R) oder (S) Stereoisomerie aufweisen kann.

In einer bevorzugten Ausführungsform der Erfindung umfasst der Eisen(III)-komplex nach Formel (1) oder (2) Z ausgewählt aus den Formeln (3) bis (6): 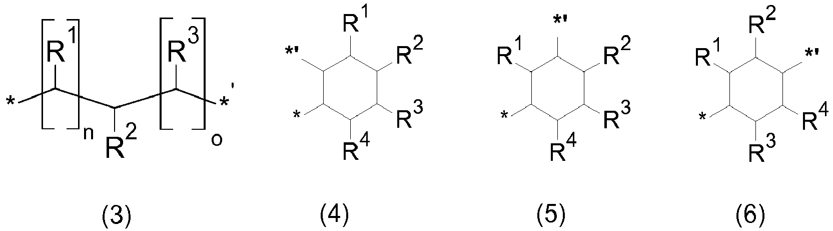 mit n, o, R¹, R², R³ und R⁴ wie zuvor definiert.

An den mit * und *' markierten Bindungsstellen der Brücken Z gemäß den obig angegebenen Formeln (4) bis (10) können die Substituenten Y¹ und Y² der Formel (1) beziehungsweise Y³ und Y⁴ der Formel (2) prinzipiell zueinander cis oder trans stehen. Vorzugsweise stehen die Gruppen Y¹ und Y² der Formel (1) beziehungsweise Y³ und Y⁴ der Formel (2) an den mit * und *' markierten Bindungsstellen jedoch zueinander in trans-Stellung. Zum Beispiel kann es sich bei Formel (4) um eine 1,2-*trans*-Cylcohexylen Gruppe handeln.

Besonders bevorzugt stehen * und * der Formeln (4) bis (10) demnach in *trans*-Stellung zueinander. In bevorzugten Ausführungsformen der Eisen(III)-komplexe nach Formel (1) oder (2) umfasst Z eine Gruppe ausgewählt aus: *N*,*N*'-1,2-*trans*-Cylcohexylen, *N,N'*-1*,*3*-trans-*Cylcohexylen, *N*,*N*'-1,4-*trans*-Cylcohexylen, *N,N'*-1,3-*trans*-Cylcopentylen, *N,N'*-1,2*-trans-*Cylcobutylen, *N*,*N'*-1,3-t*rans*-Cylcobutylen und *N*,*N'*-1,2-*trans*-Cylcopentylen.

Nach einer weiteren Ausführungsform, bei dem der Eisen(III)-komplex der Formel (1) entspricht, ist m = 2.

Ferner ist bevorzugt, wenn der Eisen(III)-komplex der Formel (1) entspricht und Y¹ und Y² für NH steht.

Bevorzugt ist weiterhin, wenn der Eisen(III)-komplex der Formel (2) entspricht und Y³ für NH und Y⁴ für OH, NH₂ oder NHR steht.

Besonders bevorzugte Ausführungsbeispiele der Eisen(III)-komplexe nach Formel (1) oder Formel (2) umfassen Verbindungen, bei denen:
- Z der Formel (4) bis (6) entspricht mit R¹ = R² = R³ = R⁴ = H, oder
- Z der Formel (5) entspricht mit R¹ = R² = R³ = H und R⁴ = CH₃, oder
- Z der Formel (5) entspricht mit R¹ = R³ = R⁴ = H und R² = CH₃, oder
- Z der Formel (8) entspricht mit R¹ = R² = H und R³ = CH₃, oder
- Z der Formel (8) entspricht mit R²= R³ = H und R¹ = CH₃.

Die nun bereitgestellten Eisen(III)-komplexe der Formeln (1) und (2) weisen einen mehrzähnigen, von Cyclohexandiamintetraessigsäure (CDTA), insbesondere trans-Cyclohexandiamintetraessigsäure (*t*CDTA) abgeleiteten Liganden auf und eignen sich beispielsweise für den Einsatz bei *in*-*vivo*-diagnostischen Verfahren, insbesondere als Kontrastmittel für die Magnetresonanztomographie (MRT).

Eisen(III)-komplexe der Formel (1) können höhere Relaxivitäten als beispielsweise Fe-*t*CDTA aufweisen und können - verglichen mit Fe-*t*CDTA (einfach negativ geladener Komplex) - eine neutrale Gesamtladung haben. Für einen neutralen pH-Wert sind daher allenfalls geringe Konzentrationen an Gegenionen notwendig. Pro Eisenatom ist daher die Osmolalität der Eisen(III)-komplexe der Formel (1) stark reduziert. Dies ist vorteilhaft für die Akuttoxizität, so dass auch höhere Dosierungen der Eisen(III)-komplexe der Formel (1) bei Verwendung in *in*-*vivo*-diagnostischen Verfahren toleriert werden. Wenn die verglichen mit Gadolinium-Komplexen geringere Relaxivität von Eisenkomplexen durch höhere Dosen ausgeglichen werden muss, so stellt eine geringere Osmolalität einen wesentlichen Vorteil der Eisen(III)-komplexe der Formel (1) gegenüber ionischen Eisenkomplexen, wie beispielsweise Fe-DTPA oder Fe-*t*CDTA, dar. Nach einer bevorzugten Ausführungsform ist beim Eisen(III)-komplex der Formel (1) m = 2. Der Eisen(III)-komplex ist demnach ein Dimer aus zwei verbrückten *t*CDTA-Einheiten.

Besonders bevorzugt sind Eisen(III)-komplexe der Formel (1-1) und (1-2):

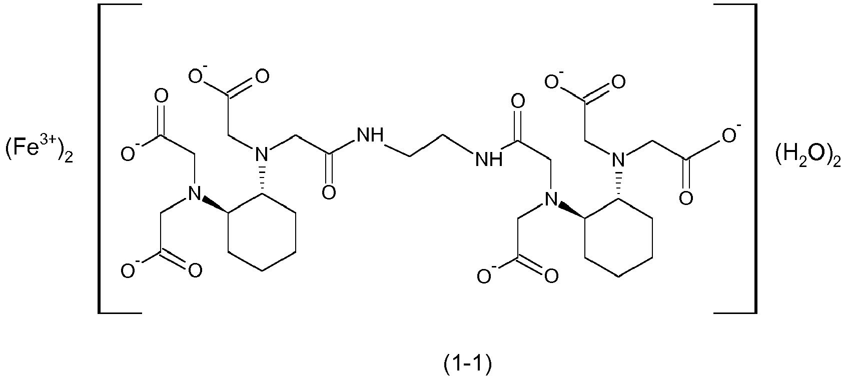

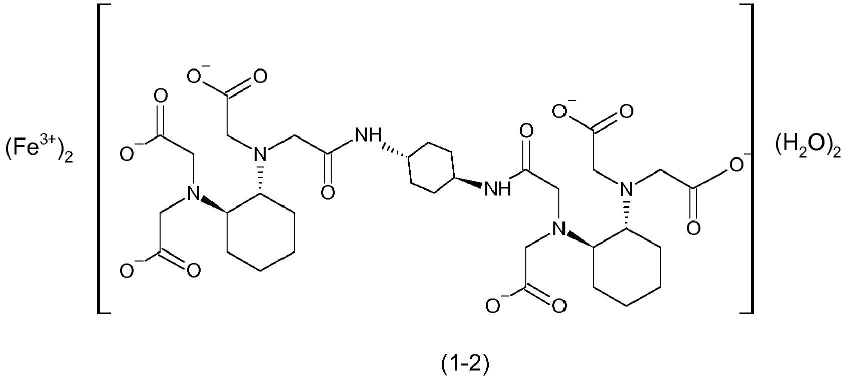

Spezielle Eisen(III)-komplexe der Formel (2) zeigen eine überraschend starke pH-Abhängigkeit der Relaxivität. Die starke pH-Abhängigkeit liegt in einem für medizinische Bildgebung besonders attraktiven pH-Wertebereich von 5 bis 8. Beispielsweise weisen Tumore meist einen leicht sauren pH-Wert im genannten Bereich auf. Es wird angenommen, dass die pH-Abhängigkeit auf eine Protonierung/Deprotonierung der endständigen Gruppe Y⁴ an der Brücke Z zurückzuführen ist.

Besonders ausgeprägt ist die pH-Wertabhängigkeit beim Eisen(III)-komplex der Formel (2-1):

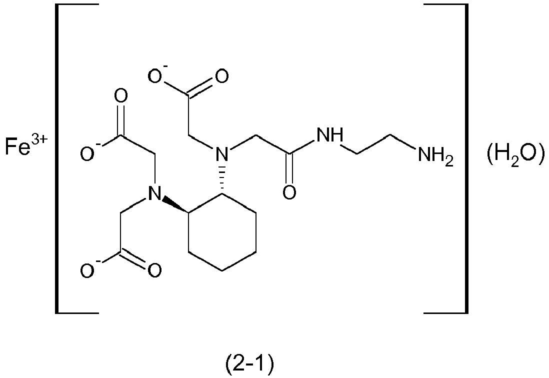

Bei niedrigem pH-Wert ist hier die endständige Aminogruppe protoniert und steht somit nicht zur Koordination für das zentrale Eisen(III)-ion zur Verfügung, so dass anstelle dessen die Koordination über Wasser als Liganden erfolgt. Bei höherem pH-Wert ist dagegen das freie Elektronenpaar am Stickstoff an der Koordination des Zentralions beteiligt, so dass Wasser aus der Koordinationsstelle verdrängt wird. Die Gegenwart eines Wasser-Liganden im Komplex führt zu einer wesentlichen Steigerung der T1- und der T2-Relaxivität, was wiederum einen verbesserten Kontrasteffekt bedingt. Tumore und Entzündungen haben zumeist einen relativ niedrigen extrazellulären pH-Wert, der somit zu einer Kontraststeigerung führen dürfte.

Diese pH-Abhängigkeit lässt sich modellhaft für den Eisen(III)-komplex (2-1) wie folgt darstellen:

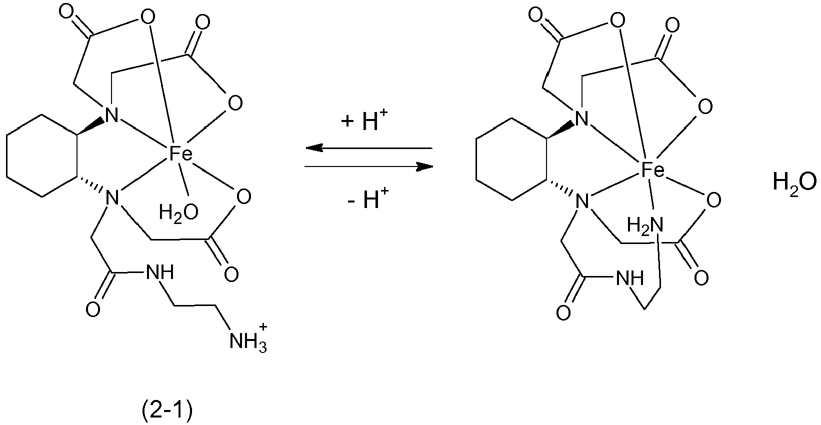

Der Effekt ließe sich beispielsweise ebenfalls für die Chemical Exchange Saturation Transfer (CEST)- bzw. Para-CEST-Bildgebung nutzen, indem die Protonen des koordinierten Wassers durch CEST abgebildet werden. Um zu konzentrationsunabhängigen pH-Werten zu kommen, kann beispielsweise zusätzlich noch das Amid-Proton durch CEST abgebildet und mit den Wasserprotonen verrechnet werden.

Bevorzugte Ausführungsformen sind Eisenkomplexe der Formeln (2-1) und (2-2).

Eine besonders bevorzugte Ausführungsform ist der Eisenkomplex der Formel (2-2):

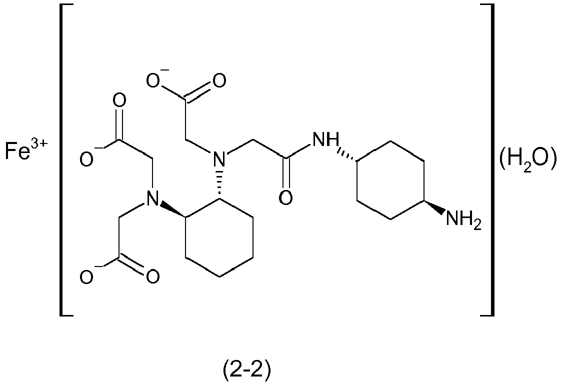

Die wenig flexible endständige Aminogruppe kann das Zentralion nicht komplexieren, wodurch eine pH-abhängige Deaktivierung verhindert wird. Die endständige Gruppe Y⁴ der Eisen(III)-komplexe der Formel (2), zum Beispiel die Aminogruppe bei den Eisen(III)-komplexen der Formeln (2-1) und (2-2), kann ferner für chemische Kopplungsreaktionen genutzt werden, um beispielsweise Biomoleküle oder andere Substanzen zu markieren und dadurch durch die MRT detektierbar zu machen. Eine Aminogruppe kann dabei in eine für die avisierte Kopplungsreaktion geeignete funktionelle Gruppe überführt werden, zum Beispiel eine Isocyanat-Gruppe.

Im Zusammenhang mit der vorliegenden Erfindung wird unter C1-C15 Alkyl ein linearer oder verzweigter Alkylrest verstanden, der die allgemeinen Formel CₙH₂ₙ₊₁ aufweist, wobei n = 1 bis 15 bedeutet. C1-C5 Alkyl umfasst beispielsweise Methyl, Ethyl, n-Propyl, 2-Propyl, n-Butyl, 2-Butyl, 2-Methylpropyl, 2-Methyl-2-propyl, n-Pentyl, 2-Pentyl, 2-Methylbutyl, 3-Methylbutyl, 2-Methyl2-butyl, 3-Methyl-2-butyl, 2,2-Dimethylpropyl und 3-Pentyl. Beispiele für Alkylreste mit n ≥ 6 umfassen n-Hexyl, n-Heptyl, n-Octyl, n-Nonyl, n-Decyl, 2-Hexyl, 2-Heptyl, 2-Octyl, 2-Nonyl, 2-Decyl, 2-Undecyl, 3-Hexyl, 3-Heptyl, 3-Octyl, 3-Nonyl, 3-Decyl, 3-Undecyl, 3-Dodecyl, 2-Methylpentyl, 2-Methylhexyl, 2-Methylheptyl, 2-Methyloctyl, 2-Methylnonyl, 2-Methyldecanyl, 2-Ethylbutyl, 2-Ethylpentyl, 2-Ethylhexyl, 2-Ethylheptyl, 2-Ethyloctyl, 2-Ethylnonyl, 2-Ethyldecanyl, 2-Methyl-2-pentyl, 2-Methyl-2-hexyl, 2-Methyl-2-heptyl, 2-Methyl-2-octyl, 2-Methyl-2-nonyl, 2-Methyl-2-decyl, 2-Methyl-2-undecyl, 3-Methyl-2-pentyl, 3-Methyl-2-hexyl, 3-Methyl-2-heptyl, 3-Methyl-2-octyl, 3-Methyl-2-nonyl, 3-Methyl-2-decyl, 3-Methyl-2-undecyl, 3-Ethyl-2-pentyl, 3-Ethyl-2-hexyl, 3-Ethyl-2-heptyl, 3-Ethyl-2-octyl, 3-Ethyl-2-nonyl, 3-Ethyl-2-decyl, 3-Ethyl-2-undecyl, 2-Methyl-3-pentyl, 4-Methyl-3-hexyl, 4-Methyl-3-heptyl, 4-Methyl-3-octyl, 4-Methyl-3-nonyl, 4-Methyl-3-decyl, 4-Methy-3-undecyl und 4-Methyl-3-dodecyl.

Im Zusammenhang mit der vorliegenden Erfindung wird ferner unter C3-C10 Cycloalkyl ein monocyclischer oder bicyclischer Cycloalkylrest mit 3 bis 15 Kohlenstoffatomen verstanden. Beispiele umfassen Cyclopropyl, Cyclobutyl, Cyclopentyl, Cyclohexyl und Cycloheptyl. Die Cycloalkylreste können ihrerseits einfach oder mehrfach mit C1-C5 Alkylresten der oben wiedergegebenen Bedeutung substituiert sein. Beispiele für derartig substituierte Cycloalkylreste umfassen 1-Methyl-1-cyclopropyl, 1-Methyl-1-cyclobutyl, 1-Methyl-1-cyclopentyl, 1-Methyl-1-cyclohexyl, 1-Methyl-1-cycloheptyl, 2-Methyl-1-cyclopropyl, 2-Methyl-1-cyclobutyl, 2-Methyl-1-cyclopentyl, 2-Methyl-1-cyclohexyl, 2-Methyl-1-cycloheptyl, 3-Methyl-1-cyclobutyl, 3-Methyl-1-cyclopentyl, 3-Methyl-1-cyclohexyl, 3-Methyl-1-cycloheptyl, 4-Methyl-1-cyclohexyl, 4-Methyl-1-cycloheptyl, 1,2-Dimethyl-1-cyclopropyl, 2,2-Dimethyl-1-cyclopropyl, 2,3-Dimethyl-1-cyclopropyl, 1,2-Dimethyl-1-cyclobutyl, 1,3-Dimethyl-1-cyclobutyl, 2,2-Dimethyl-1-cyclobutyl, 2,3-Dimethyl-1-cyclobutyl, 2,4-Dimethyl-1-cyclobutyl, 3,3-Dimethyl-1-cyclobutyl, 1,2-Dimethyl-1-cyclopentyl, 1,3-Dimethyl-1-cyclopentyl, 2,2-Dimethyl-1-cyclopentyl, 2,3-Dimethyl-1-cyclopentyl, 2,4-Dimethyl-1-cyclopentyl, 2,5-Dimethyl-1-cyclopentyl, 3,3-Dimethyl-1-cyclopentyl, 3,4-Dimethyl-1-cyclopentyl, 1,2-Dimethyl-1-cyclohexyl, 1,3-Dimethyl-1-cyclohexyl, 1,4-Dimethyl-1-cyclohexyl, 1,5-Dimethyl-1-cyclohexyl, 1,6-Dimethyl-1-cyclohexyl, 2,2-Dimethyl-1-cyclohexyl, 2,3-Dimethyl-1-cyclohexyl, 2,4-Dimethyl-1-cyclohexyl, 2,5-Dimethyl-1-cyclohexyl, 2,6-Dimethyl-1-cyclohexyl, 3,3-Dimethyl-1-cyclohexyl, 3,4-Dimethyl-1-cyclohexyl, 3,5-Dimethyl-1-cyclohexyl, 3,6-Dimethyl-1-cyclohexyl, 4,4-Dimethyl-1-cyclohexyl, 1,2,2-Trimethyl-1-cyclopropyl, 1 ,2,3-Trimethyl-1-cyclopropyl, 1,2,2-Trimethyl-1-cyclobutyl, 1,3,3-Trimethyl-1-cyclobutyl, 1,2,3-Trimethyl-1-cyclobutyl, 2,2,3-Trimethyl-1-cyclobutyl, 2,2,4-Trimethyl-1-cyclobutyl, 1,2,2-Trimethyl-1-cyclopentyl, 1,2,3-Trimethyl-1-cyclopentyl, 1,2,4-Trimethyl-1-cyclopentyl, 1,2,5-Trimethyl-1-cyclopentyl, 1,3,3-Trimethyl-1-cyclopentyl, 1,3,4-Trimethyl-1-cyclopentyl, 1,3,5-Trimethyl-1-cyclopentyl, 2,2,3-Trimethyl-1-cyclopentyl, 2,2,4-Trimethyl-1-cyclopentyl, 2,2,5-Trimethyl-1-cyclopentyl, 2,3,3-Trimethyl-1-cyclopentyl, 2,3,4-Trimethyl-1-cyclopentyl, 2,3,5-Trimethyl-1-cyclopentyl, 2,3,3-Trimethyl-1-cyclopentyl, 2,4,4-Trimethyl-1-cyclopentyl, 2,4,5-Trimethyl-1-cyclopentyl, 2,5,5-Trimethyl-1-cyclopentyl, 3,3,4-Trimethyl-1-cyclopentyl, 3,3,5-Trimethyl-1-cyclopentyl, 3,4,5-Trimethyl-1-cyclopentyl, 3,4,4-Trimethyl-1-cyclopentyl, 1,2,2-Trimethyl-1-cyclohexyl, 1,2,3-Trimethyl-1-cyclohexyl, 1,2,4-Trimethyl-1-cyclohexyl, 1,2,5-Trimethyl-1-cyclohexyl, 1,2,6-Trimethyl-1-cyclohexyl, 1,3,3-Trimethyl-1-cyclohexyl, 1,3,4-Trimethyl-1-cyclohexyl, 1,3,5-Trimethyl-1-cyclohexyl, 1,3,6-Trimethyl-1-cyclohexyl, 1,4,4-Trimethyl-1-cyclohexyl, 2,2,3-Trimethyl-1-cyclohexyl, 2,2,4-Trimethyl-1-cyclohexyl, 2,2,5-Trimethyl-1-cyclohexyl, 2,2,6-Trimethyl-1-cyclohexyl, 2,3,3-Trimethyl-1-cyclohexyl, 2,3,4-Trimethyl-1-cyclohexyl, 2,3,5-Trimethyl-1-cyclohexyl, 2,3,6-Trimethyl-1-cyclohexyl, 2,4,4-Trimethyl-1-cyclohexyl, 2,4,5-Trimethyl-1-cyclohexyl, 2,4,6-Trimethyl-1-cyclohexyl, 2,5,5-Trimethyl-1-cyclohexyl, 2,5,6-Trimethyl-1-cyclohexyl, 2,6,6-Trimethyl-1-cyclohexyl, 3,3,4-Trimethyl-1-cyclohexyl, 3,3,5-Trimethyl-1-cyclohexyl, 3,3,6-Trimethyl-1-cyclohexyl, 3,4,4-Trimethyl-1-cyclohexyl, 3,4,5-Trimethyl-1-cyclohexyl, 3,4,6-Trimethyl-1-cyclohexyl, 3,5,6-Trimethyl-1-cyclohexyl, 1,2,3,3-Tetramethyl-1-cyclopropyl, 2,2,3,3-Tetramethyl-1-cyclopropyl, 1,2,2,3-Tetramethyl-1-cyclopropyl, 1,2,2,3-Tetramethyl-1-cyclobutyl, 1,2,3,3-Tetramethyl-1-cyclobutyl, 2,2,3,3-Tetramethyl-1-cyclobutyl, 2,3,3,4-Tetramethyl-1-cyclobutyl, 1,2,2,3-Tetramethyl-1-cyclopentyl, 1,2,2,4-Tetramethyl-1-cyclopentyl, 1,2,2,5-Tetramethyl-1-cyclopentyl, 1,2,3,3-Tetramethyl-1-cyclopentyl, 1,2,3,4-Tetramethyl-1-cyclopentyl, 1,2,3,5-Tetramethyl-1-cyclopentyl, 1,2,5,5-Tetramethyl-1-cyclopentyl, 2,2,3,3-Tetramethyl-1-cyclopentyl, 2,2,3,3-Tetramethyl-1-cyclohexyl, 2,2,4,4-Tetramethyl-1-cyclohexyl, 2,2,5,5-Tetramethyl-1-cyclohexyl, 3,3,4,4-Tetramethyl-1-cyclohexyl, 3,3,5,5-Tetramethyl-1-cyclohexyl, 1-Ethyl-1-cyclopropyl, 1-Ethyl-1-cyclobutyl, 1-Ethyl-1-cyclopentyl, 1-Ethyl-1-cyclohexyl, 1-Ethyl-1-cycloheptyl, 2-Ethyl-1-cyclopropyl, 2-Ethyl-1-cyclobutyl, 2-Ethyl-1-cyclopentyl, 2-Ethyl-1-cyclohexyl, 2-Ethyl-1-cycloheptyl, 3-Ethyl-1-cyclobutyl, 3-Ethyl-1-cyclopentyl, 3-Ethyl-1-cyclohexyl, 3-Ethyl-1-cycloheptyl, 4-Ethyl-1-cyclohexyl und 4-Ethyl-1-cycloheptyl.

Die Eisen(III)-komplexe der Formeln (1) oder (2) können in in-vivo-diagnostischen Verfahren, insbesondere als Kontrastmittel für die Magnetresonanztomographie, Verwendung finden. Insbesondere kann ein Kontrastmittel für Magnetresonanztomographie, die genannten Eisen(III)-komplexe enthalten.

Für die Herstellung der Eisen(III)-komplexe werden die folgenden Liganden der Formeln (L1) oder (L2) benötigt: 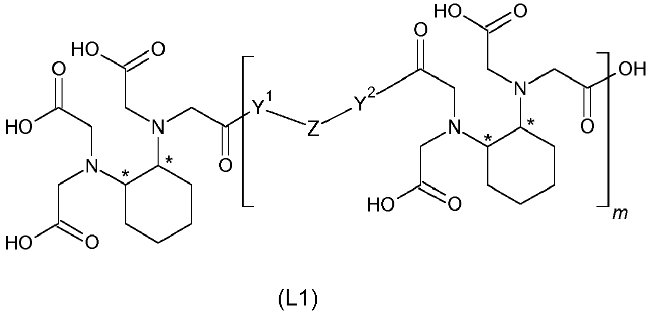 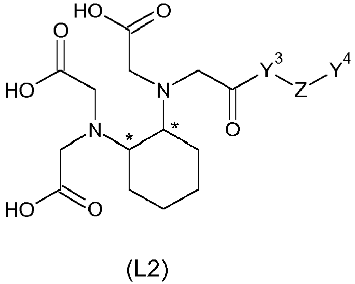 mit
m = 1 bis 5;
Y¹ und Y² unabhängig voneinander ausgewählt aus der Gruppe umfassend O, NH und NR, wobei R für C1-C15 Alkyl oder C3-C10 Cycloalkyl steht;
Y³ ausgewählt aus der Gruppe umfassend O, NH und NR, wobei R für C1-C15 Alkyl oder C3-C10 Cycloalkyl steht;
Y⁴ ausgewählt aus der Gruppe umfassend OH, OR, NH₂, NHR und NR⁵R⁶, wobei R, R⁵ und R⁶ für C1-C15 Alkyl oder C3-C10 Cycloalkyl stehen sowie R⁵ und R⁶ unabhängig voneinander ausgewählt sind;
Z bedeutet eine Brücke gemäß einer der Formeln (3) bis (10): 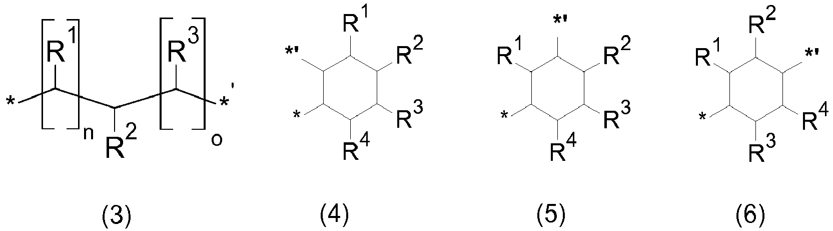 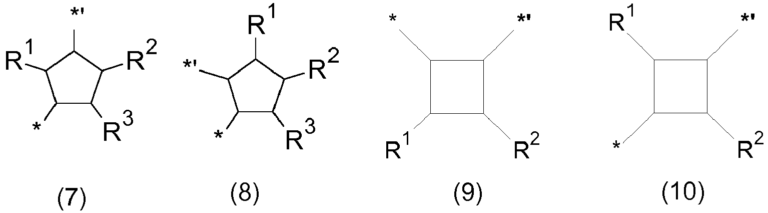 mit
   n = 0 bis 4;
   o = 0 bis 4;
   * und *' bedeuten Anbindungsstellen der Brücke Z an Y¹ und Y² der Formel (1) beziehungsweise Y³ und Y⁴ der Formel (2); und
   R¹, R², R³ und R⁴ unabhängig voneinander ausgewählt aus der Gruppe umfassend H, C1-C15 Alkyl und C3-C10 Cycloalkyl;
   ausgenommen den Liganden der Formel (L2) mit Z gleich *-CH₂CH₂-*'.

Bevorzugt sind Liganden, bei denen die beiden Substituenten an den mit * markierten asymmetrischen Kohlenstoffatomen der Formeln (L1) oder (L2) trans-ständig sind.

Wenn Z den Formeln (4) bis (10) entspricht, stehen die Gruppen Y¹ und Y² der Formel (L1) beziehungsweise Y³ und Y⁴ der Formel (L2) vorzugsweise an den mit * und *' markierten Bindungsstellen zueinander in trans-Stellung.

Bevorzugt ist ferner, wenn der Ligand der Formel (L1) entspricht und Y¹ und Y² jeweils für NH stehen.

Wenn der Ligand der Formel (L2) entspricht, steht vorzugsweise Y³ für NH und Y⁴ für OH, NH₂ oder NHR.

Besonders bevorzugt sind Liganden der Formeln (L1-1), (L1-2) und (L2-2):

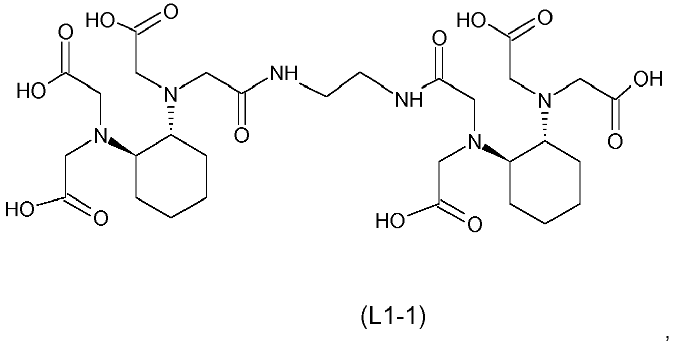

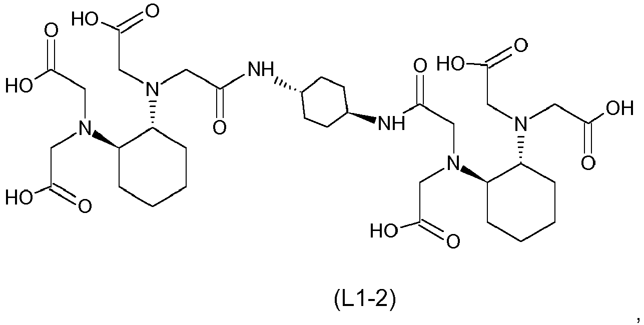

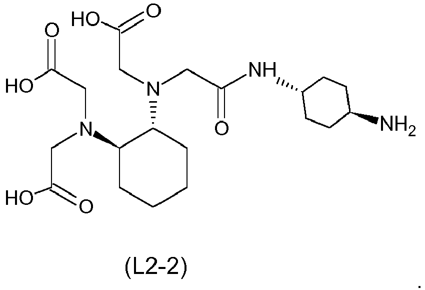

Hinsichtlich weiterer bevorzugter Ausführungsformen der Liganden der Formeln (L1) und (L2) wird auf die obigen Ausführungen zu den Eisen(III)-komplexen verwiesen.

Die Herstellung aller Eisen(III)-komplexe der Formel (2) kann analog dem folgenden allgemeinen Reaktionsschema erfolgen:

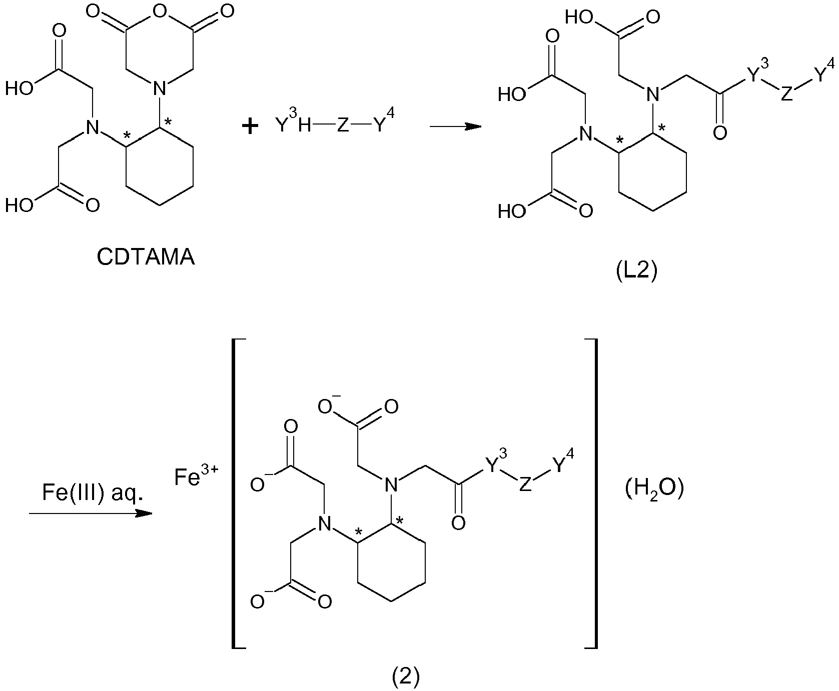

Y³, Y⁴ und Z sind wie weiter oben für die Eisen(II)-komplexe der Formel (2) bzw. Liganden der Formel (L-2) definiert. Die Synthese startet vom Anhydrid CDTAMA, vorzugsweise dem Anhydrid tCDTAMA, dessen Zugang aus der Literatur bekannt ist, siehe Gestin JF, Faivre-Chauvet A, Mease RC, et al., Nucl Med Biol, 1993; 20(6), 755-762. Ferner ist in dieser Literaturstelle auch die Umsetzung des Anhydrids tCDTAMA mit zum Beispiel Ethan-1,2-diamin (Synonym Ethylendiamin) zu N-[Methyl(2-aminoethyl)carbamid]-trans-1,2,diaminocyclohexan-N,N',N'-triessigsäure beschrieben, wobei das Anhydrid einer stark überschüssigen Lösung des Diamins in DMSO (Dimethylsulfoxid) zugesetzt wird. Der Ligand (L2) wird einer wässrigen Überschusslösung eines Fe(III)-Salzes zugesetzt und der ausfallende Komplex (2) abgetrennt. Z ist eine Gruppe der Formeln (3) bis (10) wie bereits weiter oben beschrieben.

Die Herstellung der Eisen(III)-komplexe der Formel (1) kann analog gemäß dem folgenden allgemeinen Reaktionsschema erfolgen:

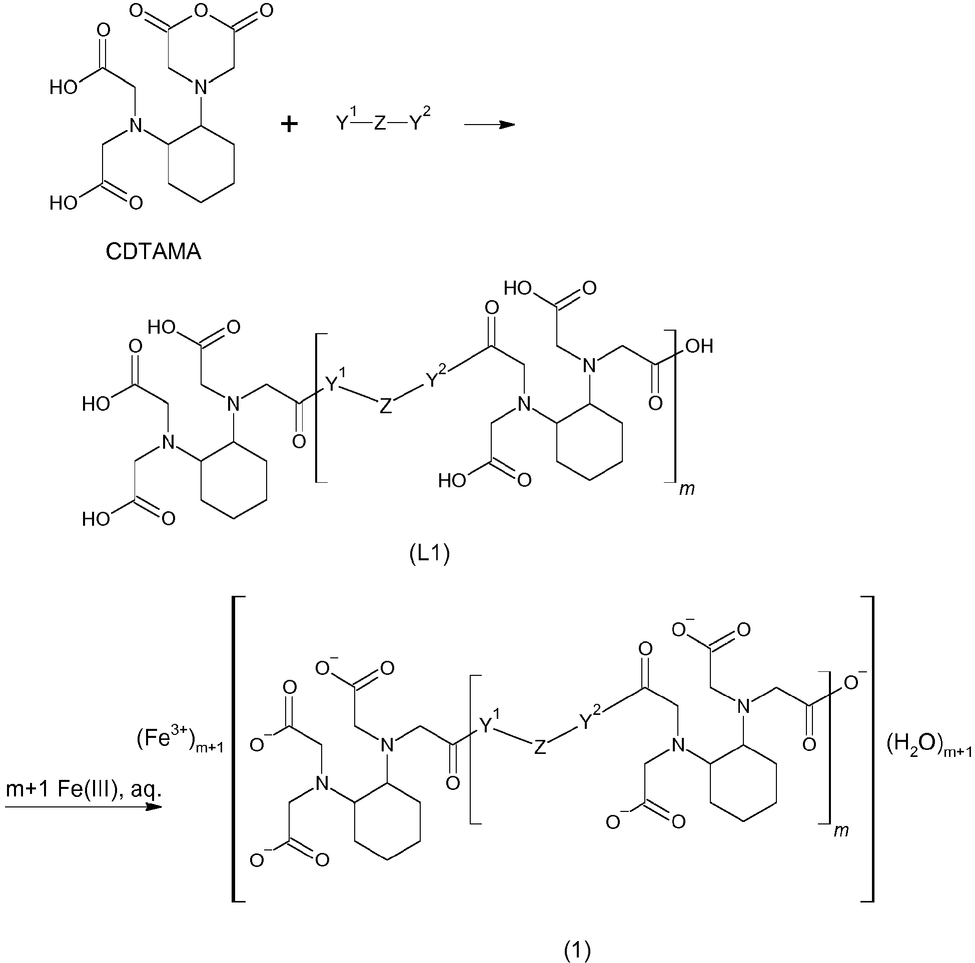

Die Synthese startet ebenfalls vom bekannten Anhydrid CDTAMA, insbesondere tCDTAMA. Nunmehr wird jedoch das Anhydrid in Lösung vorgelegt und das Kopplungsagenz, zum Beispiel ein Diamin, tropfenweise unter starkem Rühren zugesetzt. Unter diesen Bedingungen agiert zum Beispiel das Diamin als Kopplungsagenz für zwei Anhydride. Der Ligand (L1) wird einer wässrigen Überschusslösung eines Fe(III)-Salzes zugesetzt und der ausfallende Komplex (1) abgetrennt. Z ist eine Gruppe der Formeln (3) bis (10) wie bereits weiter oben beschrieben.

Gemäß einem alternativen Herstellungsverfahren der Eisen(III)-komplexe der Formeln (1) oder (2) erfolgt die Eisenbeladung der Liganden mittels Eisen(III)-hydroxid wie in der Patentanmeldung PCT/EP2019/064750 beschrieben. In diesem Verfahren wird zunächst mittels Eisen(III)-chlorid und Natriumhydroxid Eisen(III)-hydroxid hergestellt, was anschließend mit Wasser gewaschen wird. Das Eisen(III)-hydroxid wird dann direkt im Anschluss verwendet, um die Liganden mit Eisen(III) zu beladen.

Ferner kann nach einer weiteren alternativen Variante die Eisenbeladung über Eisen(III)-chlorid erfolgen (Boehm-Sturm P et al., Low-Molecular-Weight Iron Chelates May Be an Alternative to Gadolinium-based Contrast Agents for T1-weighted Contrast-enhanced MR Imaging. Radiology 2017; 286(2):537-546), die jedoch den Nachteil einer höheren NACl-Konzentration aufweist.

Gemäß noch einer weiteren Variante erfolgt die Eisenbeladung unter Verwendung von Eisen(III)-nitrat.

### Ausführungsbeispiele

### Synthese des Eisen(III)-komplexes (2-1)

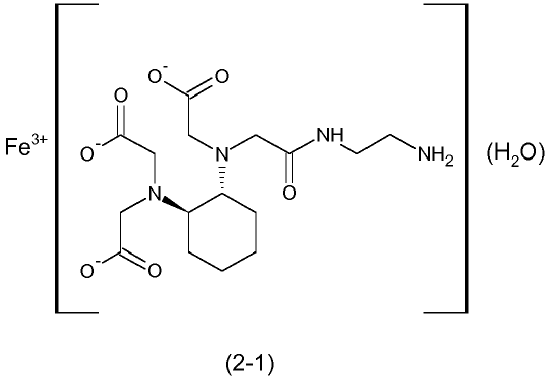

6,53 g (17,9 mmol) trans-1,2-Diaminocyclohexan-*N*,*N*,*N',N'*-tetraessigsäure (CDTA) wurden zu einer Lösung von 12,87 ml (136,2 mmol) Essigsäureanhydrid und 2,75 ml Pyridin gegeben. Nach 24-stündigem Rühren bei Raumtemperatur unter Argon wurde das Reaktionsgemisch filtriert und mit Essigsäureanhydrid, gefolgt von einem Überschuss an Ethylacetat, gewaschen. Der Feststoff wurde gesammelt und im Vakuum getrocknet, wobei 5,30 g eines weißen Pulvers (CDTA-Monoanhydrid) erhalten wurden.

CDTA-Monoanhydrid (CDTAMA) (14,4 mmol, 5 g) wurde in kleinen Portionen als Feststoff über einen Zeitraum von 6 Stunden zu einer Lösung von 19,31 ml Ethylendiamin (289 mmol) und 23,75 ml DMSO unter Argon zugegeben. Nach der Zugabe wurde die Reaktion der Mischung über Nacht bei Raumtemperatur gerührt. Die Mischung wurde dann mittels Rotationsverdampfer zu einem dicken orangen Öl konzentriert, das sich beim Stehen verfestigte. Der Rückstand wurde in Methanol gelöst und bildete ein Präzipitat bei Raumtemperatur, das dann mit Methanol gewaschen wurde. Es wurden 3,98 g 2,2'-({(1*R*,2*R*)-2-[{2-[(2-aminoethyl)amino]-2-oxoethyl}(carboxymethyl)amino]cyclohexyl}-azanediyl)diessigsäure (L2-1) als weißer Feststoff erhalten.

2 g von Eisen(III)-nitrat Nonahydrat wurde in 10 ml Wasser gelöst und filtriert. Eine Mischung aus 5 ml Ammoniumhydroxid-Lösung (28-30% in Wasser) und 5 ml Wasser wurde tropfenweise in die Eisen(III)-Nitratlösung gegeben. Das entstandene unlösliche Eisen(III)-hydroxid wurde durch Filtration (Büchnertrichter) gesammelt und mehrmals mit Wasser gewaschen.

1,9 g L2-1 wurde in 4 ml Wasser gelöst. Das Eisen(III)-hydroxid wurde hinzugegeben und das Volumen der Lösung auf 10 ml mit Wasser aufgefüllt. Die Lösung wurde ca. 2 h bei 95 °C erhitzt, dabei gerührt. Danach wurde die Lösung abgekühlt und filtriert (0,2 µm) und danach das Volumen durch Erhitzen auf 2 ml reduziert. Der Eisenkomplex wurde durch Zugabe von Aceton (ca. 30 ml) ausgefällt, zentrifugiert und getrocknet. Eisen(III)-komplex 2-1 wurde in Wasser gelöst und der pH mit Meglumin auf 7,3-7,4 eingestellt.

### Synthese des Liganden (L1-1)

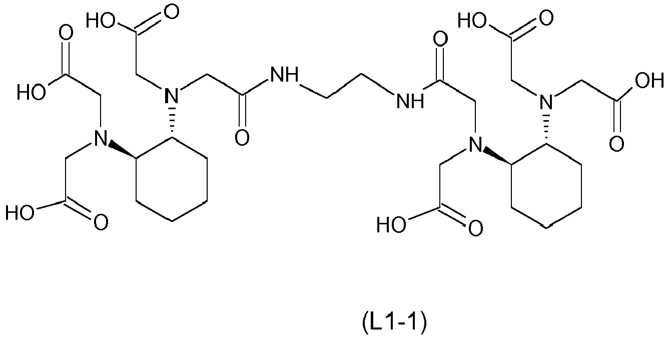

6,42 g (17,6 mmol) trans-1,2-Diaminocyclohexan-*N*,*N*,*N'*,*N'*-tetraessigsäure (*t*CDTA) wurden zu einer Lösung von 12,66 ml (134,0 mmol) Essigsäureanhydrid und 2,71 ml Pyridin gegeben Nach Rühren für 24 h bei Raumtemperatur unter Argon wurde das Reaktionsgemisch filtriert und mit Essigsäureanhydrid, gefolgt von einem Überschuss an Ethylacetat, gewaschen. Der Feststoff wurde gesammelt, im Vakuum getrocknet und ergab 5,16 g eines weißen Pulvers.

### N-[Methyl-(2-aminoethyl)carbamid]-trans-1,2-diaminocyclohexan-N,N',N'-triessigsäure

CDTAMA (12,5 mmol, 4,33 g) wurde in kleinen Portionen als Feststoff über einen Zeitraum von 6 h zu einer Lösung von 0,25 ml Ethylendiamin (3,75 mmol), 36,99 ml DMSO und 4,04 ml (50 mmol) Pyridin unter Argon zugegeben. Nach der Zugabe wurde das Reaktionsgemisch über Nacht bei Raumtemperatur gerührt. Nach Trocknung im Rotationsverdampfer wurde der Rückstand mit reichlich Ether und Ethanol gewaschen und danach durch Säulenchromatographie mit Sephadex G-10-Gel gereinigt. Die Produktfraktionen wurden mit HPLC analysiert, gesammelt und dann lyophilisiert.

### Synthese des Vergleichsbeispiels [Fe(III)tCDTA]⁻

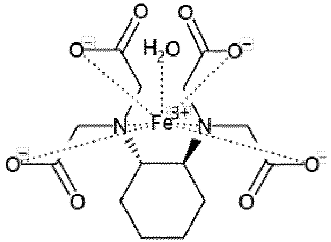

20 g von Eisen(III)-nitrat-Nonahydrat wurde in 100 ml Wasser gelöst und filtriert. Eine Mischung aus 50 ml Ammoniumhydroxid-Lösung (28-30% in Wasser) und 50 ml Wasser wurde tropfenweise in die Eisen(III)-nitratlösung gegeben. Das entstandene unlösliche Eisen(III)-hydroxid wurde durch Filtration (Büchnertrichter) gesammelt und mehrmals mit Wasser gewaschen.

17,1 g *t*CDTA wurde gelöst in 40 ml Wasser. Das Eisen(III)-hydroxid wurde hinzugegeben und das Volumen der Lösung auf 100 ml mit Wasser aufgefüllt. Die Lösung wurde ca. 2 h bei 95 °C erhitzt, dabei gerührt. Danach wurde die Lösung abgekühlt und filtriert (0,2 µm) und danach das Volumen durch Erhitzen auf 20 ml reduziert. Die Eisenkomplexe wurden durch Zugabe von Aceton (ca. 300 ml) ausgefällt, zentrifugiert und getrocknet. Fe-*t*CDTA wurde in Wasser gelöst und der pH mit Meglumin auf 7,3-7,4 eingestellt. (Eisenkonzentration 38 mg/ml; 0,68 M)

### Synthese des Liganden tCDTA-mono-trans-1,4-Diaminocyclohexan (L2-2)

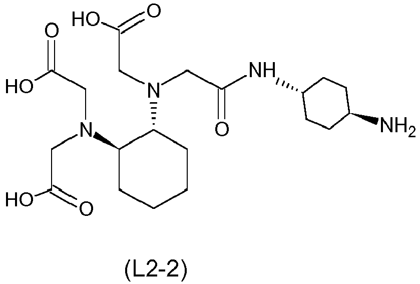

6,53 g (17,9 mmol) trans-1,2-Diaminocyclohexan-*N,N,N*',*N*'-tetraessigsäure (CDTA) wurden zu einer Lösung von 12,87 ml (136,2 mmol) Essigsäureanhydrid und 2,75 ml Pyridin gegeben. Nach Rühren für 24 h bei Raumtemperatur unter Argon wurde das Reaktionsgemisch filtriert und mit Essigsäureanhydrid, gefolgt von Ethylacetat, gewaschen. Der Feststoff wurde gesammelt, im Vakuum getrocknet und ergab 5,3 g *N*-[Methyl-(2-aminoethyl)carbamid]-trans-1,2-diaminocyclohexan-*N*,*N*',*N*'-Triessigsäure (81,2%, CDTAMA) als weißes Pulver.

Unter Argon wurden über einen Zeitraum von 6 h portionsweise 2,4 g *N*-[Methyl-(2-aminoethyl)carbamid]-trans-1,2-diaminocyclohexan-*N*,*N*',*N*'-Triessigsäure (6,93 mmol, CDTAMA) in eine Lösung aus 3,17 g trans-1,4-Diamonicyclohexan (27,7 mmol) in 18,24 mL DMSO bei 110°C zugegeben und gelöst. Nach der vollständigen Zugabe wurde das Gemisch über Nacht bei Raumtemperatur gerührt. Das Gemisch wurde unter reduziertem Druck eingeengt, in absolutem Methanol aufgenommen und über 2 Tage bei Raumtemperatur auskristallisiert. Der Feststoff wurde mit Methanol gewaschen. Es wurden 1,89 g L2-2 (79%) als weißer Feststoff erhalten.

### Synthese des Liganden trans-1,4-Diaminocyclohexan-tCDTA-Dimers (L1-2)

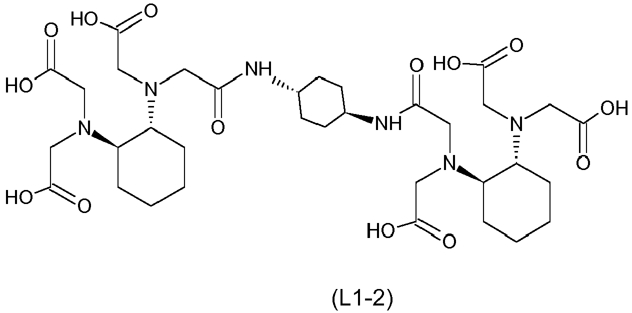

6,42 g (17,6 mmol) trans-1,2-Diaminocyclohexan-*N*,*N,N*',*N'*-tetraessigsäure (CDTA) wurden zu einer Lösung von 12,66 ml (134,0 mmol) Essigsäureanhydrid und 2,71 ml Pyridin gegeben. Nach Rühren für 24 h bei Raumtemperatur unter Argon wurde das Reaktionsgemisch filtriert und mit Essigsäureanhydrid, gefolgt von Ethylacetat, gewaschen. Der Feststoff wurde im Vakuum getrocknet (CDTAMA; weißes Pulver).

Zu einer Lösung von trans-1,4-Diaminocyclohexan (7,4 mmol, 0,85 g) in 4,82 ml (59,6 mmol) Pyridin und 58,80 ml DMSO wurde CDTAMA (17,9 mmol, 5,16 g) zugegeben und über Nacht unter Argon bei Raumtemperatur gerührt. Die Mischung wurde am Rotationsverdampfer getrocknet. Der weiße Rückstand wurde mit absolutem Ethanol gelöst, dann der Überstand vom Rückstand abgenommen und dieser erneut mittels Rotationsverdampfer getrocknet. Die resultierenden Kristalle ergaben in der HPLC einen einzelnen Produktpeak.

### Bestimmung der T1- und T2-Relaxivität

Figur 1 zeigt die pH-Abhängigkeit der T1- und T2-Relaxivität des Eisen(III)-komplexes der Formel (2-1) in Wasser (0,94 T NMR relaxometer, Bruker Minispec mq 40, Karlsruhe, Germany). Hieraus ist zu entnehmen, dass sowohl die T1-Relaxivität (Fig.1A) als auch die T2-Relaxivität (Fig. 1B) des Eisen(III)-komplexes der Formel (2-1) einem sigmoidalen Anstieg der der Relaxivität mit sinkendem pH Wert folgt, dessen maximale Änderung zwischen pH 6 und pH 7 liegt. Bei pH < 6 wird eine maximale T1-Relaxivität von 1,7 I.mmol⁻¹·s⁻¹, bzw. T2-Relaxivität von 1,9 I·mmol⁻¹·s⁻¹ erreicht.

Für die nachfolgend aufgeführten T1-Relaxivitätsmessungen wurden als Lösungsmittel für die Metallkomplexe Reinstwasser und Fetales Kälberserum (englisch: fetal bovine serum, FBS, pH 7,7) verwendet. Die Relaxivitätsmessungen bei unterschiedlichen magnetischen Feldstärken wurden an folgenden Geräten durchgeführt: 0,94 T mit einem NMR relaxometer (Bruker Minispec mq 40, Germany), 1,5 T mit einem SIEMENS MAGNETOM Sonata MRT, 3 T mit einem SIEMENS MAGNETOM Lumina MRT und 7 T mit einem Bruker Pharmascan Kleintier-MRT. Alle weiteren Parameter können Tabelle 1 entnommen werden.

| **Tabelle 1:** T1-Relaxivität (r1) von Eisen(II)komplexen mit ausgewählten Liganden in I.mmol⁻¹·s⁻¹ pro Metall-Ion bestimmt bei 0,94 T, 1,5 T, 3,0 T und 7,0 T. | | | | | |
|---|---|---|---|---|---|
| **Feldstärke T (Temperatur)** | **Lösungsmittel** | **T1-Relaxivität [I·mmol⁻¹·s⁻¹]** | | | |
| | | **pro Metall-Ion** | | | **pro Molekül** |
| | | **Vergleichsbeispiel [Fe(III)*t*CDTA]⁻** | **(2-2)** | **(1-2)** | **(1-2)** |
| **0,94 (37°C)** | **Wasser** | 1,56 | 1,92 | 1,99 | 3,98 |
| | **FBS** | 1,99 | 2,01 | 2,18 | 4,36 |
| **1,5 (23°C)** | **Wasser** | - | 2,27 | 2,75 | 5,5 |
| | **FBS** | - | 2,74 | 3,26 | 6,52 |
| **3,0 (37°C)** | **Wasser** | 2,07 | 2,64 | 2,99 | 5,98 |
| | **FBS** | 2,35 | 3,06 | 3,39 | 6,78 |
| **7,0 (37°C)** | **Wasser** | 1,87 | 2,38 | 2,65 | 5,3 |
| | **FBS** | 2,71 | 2,61 | 3,23 | 6,46 |

Die Ergebnisse der T1-Relaxivitätsmessungen zeigen (vgl. Tabelle 1), dass die erfindungsgemäßen Ausführungsbeispiele (**2-2**) und (**1-2**) insbesondere bei den klinisch besonders relevanten Feldstärken von 1,5 T und 3 T deutlich höhere Relaxivitäten im Vergleich zu [Fe(III)*t*CDTA]⁻ aufweisen. Für das Dimer (**1-2**) ist die Relaxivität bezogen auf das Gesamtkomplexmolekül, was für die Bioverteilung relevant sein kann, besonders hoch. Im Hinblick auf die Verwendung als Kontrastmittel für die Magnetresonanztomographie in *in-vivo*-diagnostischen Verfahren kann dies zu einer verbesserten Bildgebung führen.

## Patentansprüche

1. Eisen(III)-komplex nach Formel (1) oder (2): 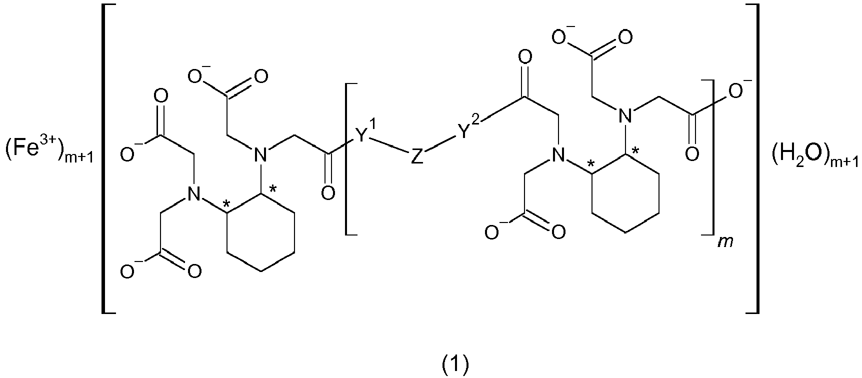 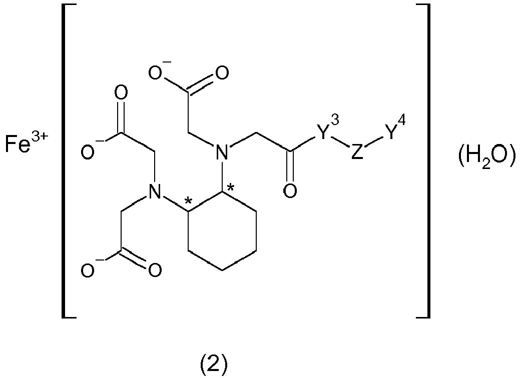 mit
m = 1 bis 5;
Y¹ und Y² unabhängig voneinander ausgewählt aus der Gruppe umfassend O, NH und NR, wobei R für C1-C15 Alkyl oder C3-C10 Cycloalkyl steht;
Y³ ausgewählt aus der Gruppe umfassend O, NH und NR, wobei R für C1-C15 Alkyl oder C3-C10 Cycloalkyl steht;
Y⁴ ausgewählt aus der Gruppe umfassend OH, OR, NH₂, NHR und NR⁵R⁶, wobei R, R⁵ und R⁶ für C1-C15 Alkyl oder C3-C10 Cycloalkyl stehen sowie R⁵ und R⁶ unabhängig voneinander ausgewählt sind;
Z bedeutet eine Brücke gemäß einer der Formeln (3) bis (10): 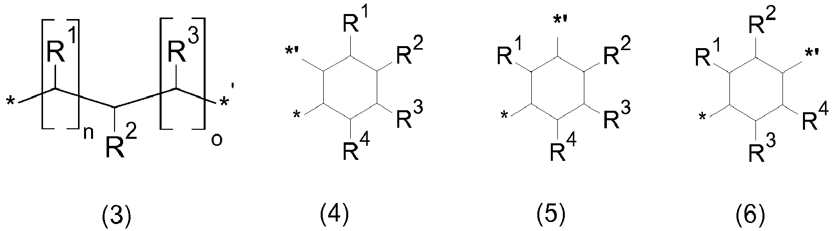 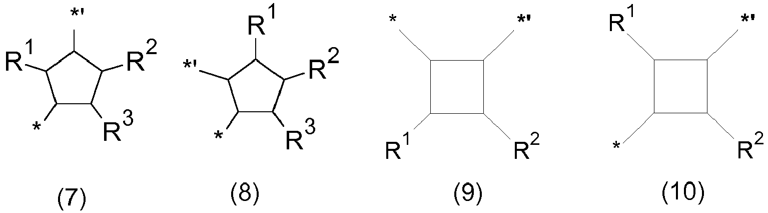 mit
n = 0 bis 4;
o = 0 bis 4;
* und *' bedeuten Anbindungsstellen der Brücke Z an Y¹ und Y² der Formel (1) beziehungsweise Y³ und Y⁴ der Formel (2); und
R¹, R², R³ und R⁴ unabhängig voneinander ausgewählt aus der Gruppe umfassend H, C1-C15 Alkyl und C3-C10 Cycloalkyl.

2. Eisen(III)-komplex nach Anspruch 1, bei dem die beiden Substituenten an den mit * markierten asymmetrischen Kohlenstoffatomen der Formeln (1) oder (2) trans-ständig angeordnet sind.

3. Eisen(III)-komplex nach Anspruch 1 oder 2, bei dem Z den Formeln (4) bis (10) entspricht und die Gruppen Y¹ und Y² der Formel (1) beziehungsweise Y³ und Y⁴ der Formel (2) an den mit * und *' markierten Bindungsstellen zueinander in trans-Stellung stehen.

4. Eisen(III)-komplex nach einem der vorherigen Ansprüche, bei dem der Eisen(III)-komplex der Formel (1) entspricht und m = 2 ist.

5. Eisen(III)-komplex nach einem der vorherigen Ansprüche, bei dem der Eisen(III)-komplex der Formel (1) entspricht und Y¹ und Y² für NH steht.

6. Eisen(III)-komplex nach einem der vorherigen Ansprüche, bei dem der Eisen(III)-komplex der Formel (2) entspricht und Y³ für NH und Y⁴ für OH, NH₂ oder NHR steht.

7. Eisen(III)-komplexe der Formeln (1-1), (1-2), (2-1) oder (2-2):

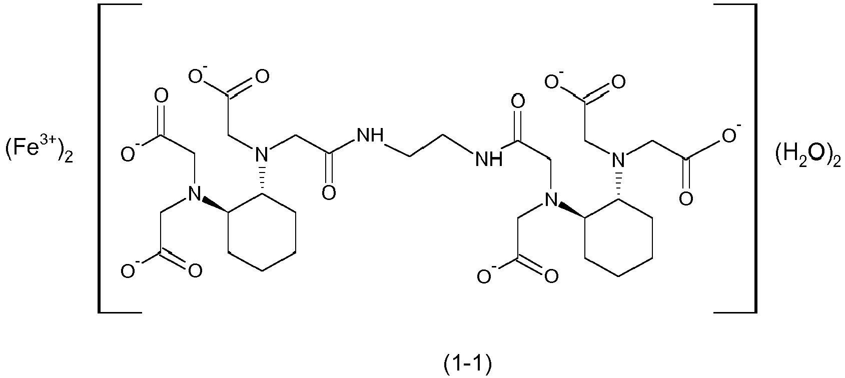

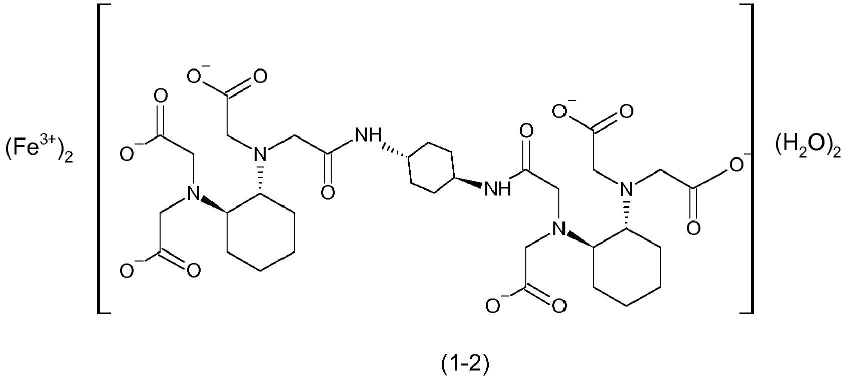

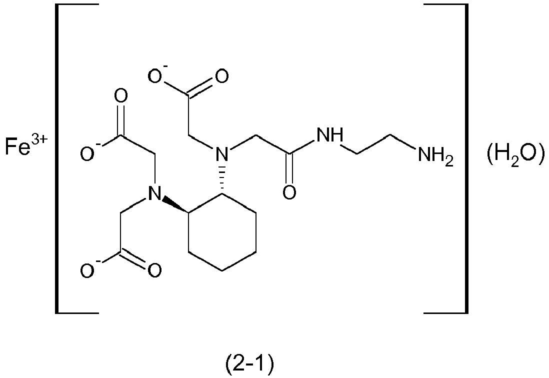

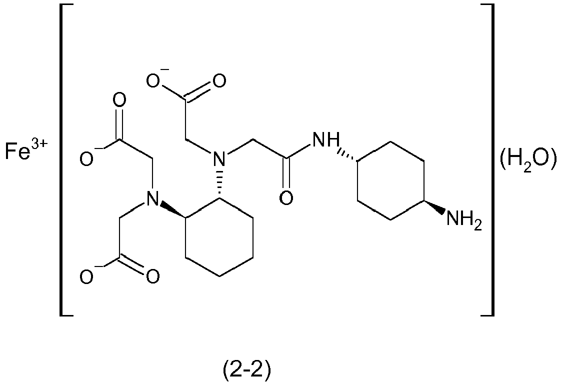

8. Verwendung eines Eisen(III)-komplexes nach einem der vorhergehenden Ansprüche als Kontrastmittel für die Magnetresonanztomographie.

9. Kontrastmittel für die Magnetresonanztomographie, enthaltend einen Eisen(III)-komplex nach einem der Ansprüche 1 bis 7.

10. Ligand nach Formel (L1) oder (L2): 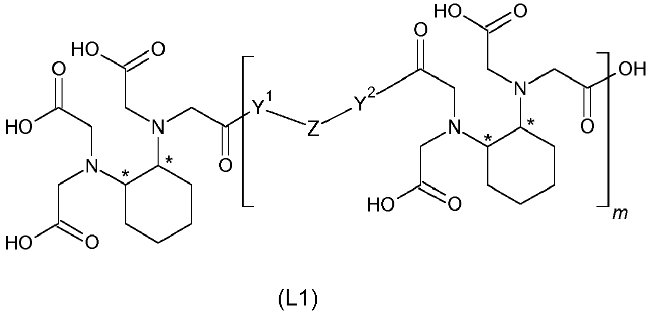 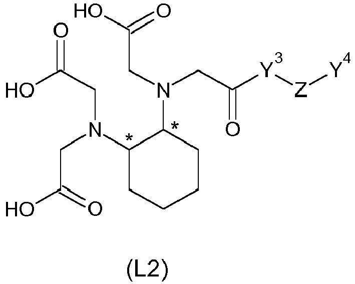 mit
m = 1 bis 5;
Y¹ und Y² unabhängig voneinander ausgewählt aus der Gruppe umfassend O, NH und NR, wobei R für C1-C15 Alkyl oder C3-C10 Cycloalkyl steht;
Y³ ausgewählt aus der Gruppe umfassend O, NH und NR, wobei R für C1-C15 Alkyl oder C3-C10 Cycloalkyl steht;
Y⁴ ausgewählt aus der Gruppe umfassend OH, OR, NH₂, NHR und NR⁵R⁶, wobei R, R⁵ und R⁶ für C1-C15 Alkyl oder C3-C10 Cycloalkyl stehen sowie R⁵ und R⁶ unabhängig voneinander ausgewählt sind;
Z bedeutet eine Brücke gemäß einer der Formeln (3) bis (10): 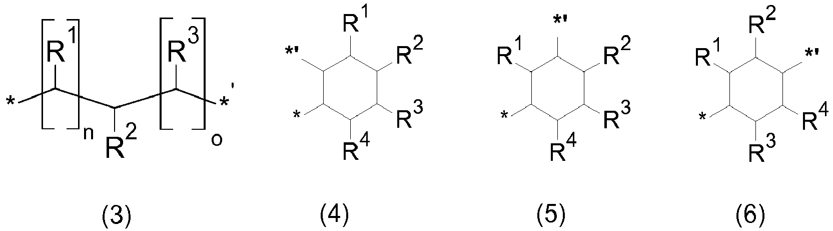 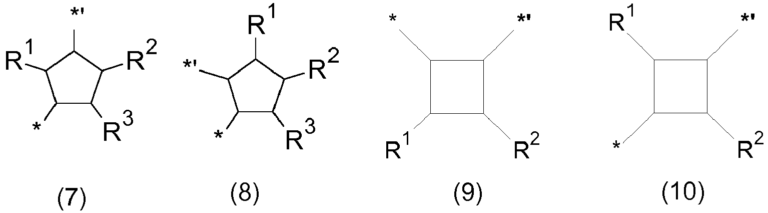 mit
n = 0 bis 4;
o = 0 bis 4;
* und *' bedeuten Anbindungsstellen der Brücke Z an Y¹ und Y² der Formel (1) beziehungsweise Y³ und Y⁴ der Formel (2); und
R¹, R², R³ und R⁴ unabhängig voneinander ausgewählt aus der Gruppe umfassend H, C1-C15 Alkyl und C3-C10 Cycloalkyl;
ausgenommen den Liganden der Formel (L2) mit Z gleich *-CH₂CH₂-*'.

11. Ligand nach Anspruch 10, bei dem die beiden Substituenten an den mit * markierten asymmetrischen Kohlenstoffatomen der Formeln (L1) oder (L2) trans-ständig sind.

12. Ligand nach Anspruch 10 oder 11, bei dem Z den Formeln (4) bis (10) entspricht und die Gruppen Y¹ und Y² der Formel (L1) beziehungsweise Y³ und Y⁴ der Formel (L2) an den mit * und *' markierten Bindungsstellen zueinander in trans-Stellung stehen.

13. Ligand nach einem der Ansprüche 10 bis 12, bei dem der Ligand der Formel (L1) entspricht und Y¹ und Y² für NH steht.

14. Ligand nach einem der Ansprüche 10 bis 13, bei dem der Ligand der Formel (L2) entspricht und Y³ für NH und Y⁴ für OH, NH₂ oder NHR steht.

15. Ligand nach Anspruch 10, ausgewählt aus:
